# EUROPEAN PATENT SPECIFICATION

(11) **EP 3 337 524 B1**
(45) Date of publication and mention of the grant of the patent: **10.03.2021**
(21) Application number: 15902462.9
(22) Date of filing: 03.12.2015
(51) Int. Cl.: A61L 27/36, A61L 27/34, A61L 27/54

(54) **EXTRACELLULAR MATRIX PROSTHESES FOR TREATING DAMAGED BIOLOGICAL TISSUE**
EXTRAZELLULÄRE MATRIXPROTHESEN ZUR BEHANDLUNG VON GESCHÄDIGTEM BIOLOGISCHEM GEWEBE
PROTHÈSES DE MATRICE EXTRACELLULAIRE POUR LE TRAITEMENT DE TISSU BIOLOGIQUE ENDOMMAGÉ

(30) Priority: 21.08.2015 US 201514832109
(43) Date of publication of application: 27.06.2018
(73) Proprietor: Cormatrix Cardiovascular, Inc., Roswell, GA 30076 (US)
(72) Inventor: MATHENY, Robert, G., Norcross, GA 30096 (US)
(74) Representative: Witthoff Jaekel Steinecke Patentanwälte PartG mbB
(86) International application number: PCT/US2015/063616
(87) International publication number: WO 2017/034600

(56) References cited:
- WO-A1-2010/096458
- US-A1- 2010 266 654
- US-A1- 2014 171 908
- US-A1- 2014 205 565
- US-A1- 2014 370 116
- US-A1- 2015 100 115
- US-A1- 2015 100 115

## Description

### FIELD OF THE INVENTION

The present invention relates to a biomaterial composition for use in treating damaged cardiovascular tissue as defined in the claims.

### BACKGROUND OF THE INVENTION

As is well known in the art, tissue prostheses (or grafts) are often employed to treat or replace damaged or diseased biological tissue. However, despite the growing sophistication of medical technology, the use of grafts to treat or replace damaged biological tissue remains a frequent and serious problem in health care. The problem is often associated with the materials employed to construct the grafts.

As is also well known in the art, the optimal graft material should be chemically inert, non-carcinogenic, capable of resisting mechanical stress, capable of being fabricated in the form required, and sterilizable. Further, the material should be resistant to physical modification by tissue fluids, and not excite an inflammatory reaction, induce a state of allergy or hypersensitivity, or, in some cases, promote visceral adhesions. See, e.g., Jenkins, et al., Surgery, vol. 94(2), pp. 392-398 (1983).

US 2015/100115 A1 describes vascular grafts comprising a multi-sheet laminate structure comprising ECM sheet members, wherein some of the sheet members comprise a top ECM-mimicking biomaterial composition coated surface, which is in contact with a non-coated surface of a second ECM sheet member. WO 2010/096458 A1 describes compositions and methods for treating or preventing cardiac arrhythmia in a subject utilizing an extracellular matrix derived from mammalian sources. US 2014/171908 A1 describes devices, systems and methods for site specific delivery of pharmacological agents and compositions to damaged and diseased cardiovascular tissue; and means for implanting and using the delivery systems to enable delivery of pharmacological agents and compositions to cardiovascular tissue. US 2014/370116 A1 describes s composition for reconstruction, replacement or repair of damaged or diseased biological tissue comprising an extracellular matrix (ECM) composition that includes an ECM scaffold component and a bioactive agent component.

Various materials and/or structures have thus been employed to construct grafts that satisfy the aforementioned optimal characteristics, including tantalum gauze, stainless mesh, Dacron®, Orlon®, Fortisan®, nylon, knitted polypropylene (e.g., Marlex®), microporous expanded-polytetrafluoroethylene (e.g., Gore-Tex®), Dacron reinforced silicone rubber (e.g., Silastlc®), polyglactin 910 (e.g., Vicryl®), polyester (e.g., Mersilene®), polyglycolic acid (e.g., Dexon®), processed sheep dermal collagen, crosslinked bovine pericardium (e.g., Peri-Guard®), and preserved human dura (e.g., Lyodura®).

As discussed in detail below, although some of the noted graft materials satisfy some of the aforementioned optimal characteristics, few, if any, satisfy all of the optimal characteristics.

The major advantages of metallic meshes, e.g., stainless steel meshes, are that they are inert, resistant to infection and can stimulate fibroplasia. Several additional disadvantages are fragmentation, which can, and in many instances will, occur after the first year of administration, and the lack of malleability.

Synthetic meshes have the advantage of being easily molded and, except for nylon, retain their tensile strength in or on the body. In European Patent No. 91122196.8 a triple-layer vascular prosthesis is disclosed that utilizes non-resorbable synthetic mesh as the center layer. The synthetic textile mesh layer is used as a central frame to which layers of collagenous fibers are added, resulting in the tri-layered prosthetic device.

There are several drawbacks and disadvantages associated with non-resorbable synthetic mesh. Among the major disadvantages are the lack of inertness, susceptibility to infection, and interference with wound healing.

In contrast to non-resorbable synthetic meshes, absorbable synthetic meshes have the advantage of impermanence at the deployment site, but often have the disadvantage of loss of mechanical strength (as a result of dissolution by the host) prior to adequate cell and tissue ingrowth.

The most widely used graft material for abdominal wall replacement and for reinforcement during hernia repairs is Marlex®, i.e. polypropylene. A major disadvantage associated with polypropylene mesh grafts is that with scar contracture, polypropylene mesh grafts become distorted and separate from surrounding normal tissue.

Gore-Tex®, i.e. polytetrafluoroethylene, is currently believed to be the most chemically inert graft material. However, a major problem associated with the use of polytetrafluoroethylene is that in a contaminated wound it does not allow for any macromolecular drainage, which limits treatment of infections.

Extracellular matrix (ECM) is also often employed to construct tissue prostheses and particulate structures. Illustrative are the grafts disclosed in U.S. Pat. Nos. 3,562,820 (tubular, sheet and strip grafts formed from submucosa adhered together by use of a binder paste, such as a collagen fiber paste, or by use of an acid or alkaline medium) and 4,902,508 (a three layer tissue graft composition derived from small intestine comprising tunica submucosa, the muscularis mucosa, and stratum compactum of the tunica mucosa), and the particulate structures disclosed in Co-Pending App. No. 14/566,404.

Although many of the ECM based tissue prostheses or grafts satisfy many of the aforementioned optimal characteristics, when the ECM graft comprises two or more sheets, i.e. a multi-sheet laminate, such as disclosed in Co-pending Application No. 14/031,423, the laminate structure can, and in some instances will, delaminate.

Thus, readily available, versatile vascular grafts that are not prone to calcification, thrombosis, intimal hyperplasia and delamination would fill a substantial and growing clinical need.

### SUMMARY OF THE INVENTION

The present invention is defined in the appended claims.

In particular, the invention provides a biomaterial composition for use in treating damaged cardiovascular tissue, comprising:
a plurality of particulate structures and a liquid buffer solution, each of said plurality of particulate structures comprising a particulate component that is fully encased in a biomaterial coating,
said particulate component comprising a size in the range of 20-300 microns,
said particulate component further comprising an extracellular matrix (ECM) composition, said ECM composition comprising acellular ECM from a mammalian tissue source,
said biomaterial coating comprising poly(glycerol sebacate) (PGS).

In an embodiment the biomaterial composition for use according to the invention is provided, wherein said liquid buffer solution comprises saline.

In an embodiment the biomaterial composition for use according to the invention is provided, wherein said mammalian tissue source is selected from the group consisting of small intestine submucosa (SIS), urinary bladder submucosa (UBS), urinary basement membrane (UBM), liver basement membrane (LBM), stomach submucosa (SS), mesothelial tissue, placental tissue and cardiac tissue.

In an embodiment the biomaterial composition for use according to the invention is provided, wherein said ECM composition further comprises a growth factor selected from the group consisting of a basic fibroblast growth factor (bFGF), transforming growth factor-beta (TGF-β) and vascular epithelial growth factor (VEGF).

### BRIEF DESCRIPTION OF THE DRAWINGS

Further features and advantages will become apparent from the following and more particular description of the preferred embodiments of the invention, as illustrated in the accompanying drawings, and in which like referenced characters generally refer to the same parts or elements throughout the views, and in which:
FIGURE 1 is a perspective view of an ECM member;
FIGURE 2 is front plan view of the ECM member shown in FIGURE 1;
FIGURE 3A is a front plan view of a multi-sheet or layer pre-laminate structure;
FIGURE 3B is a front plan view of a multi-sheet tissue prosthesis formed from the pre-laminate structure shown in FIGURE 3A;
FIGURE 4 is perspective view of another a multi-sheet laminate tissue prosthesis;
FIGURE 5 is a side or edge plan view of the tissue prosthesis shown in FIGURE 4;
FIGURE 6 is perspective view of another embodiment of a multi-sheet laminate tissue prosthesis;
FIGURE 7 is a side or edge plan view of the tissue prosthesis shown in FIGURE 6; and
Figure 8 is a front sectional view of a particulate structure, in accordance with the invention.

### DETAILED DESCRIPTION OF THE PREFERRED EMBODIMENT

Before describing the present invention in detail, it is to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

As used in this specification and the appended claims, the singular forms "a, "an" and "the" include plural referents unless the content clearly dictates otherwise. Thus, for example, reference to "a pharmacological agent" includes two or more such agents and the like.

Further, ranges can be expressed herein as from "about" or "approximately" one particular value, and/or to "about" or "approximately" another particular value. When such a range is expressed, another embodiment includes from the one particular value and/or to the other particular value. Similarly, when values are expressed as approximations, by use of the antecedent "about" or "approximately", it will be understood that the particular value forms another embodiment. It will be further understood that the endpoints of each of the ranges are significant both in relation to the other endpoint, and independently of the other endpoint.

It is also understood that there are a number of values disclosed herein, and that each value is also herein disclosed as "about" or "approximately" that particular value in addition to the value itself. For example, if the value "10" is disclosed, then "approximately 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed then "less than or equal to 10" as well as "greater than or equal to 10" is also disclosed.

### Definitions

The terms "graft" and "endograft" are used interchangeably herein, and mean and include a structure that is configured for implantation in a cardiovascular structure, e.g., a cardiovascular vessel.

The term "particulate", as used herein, means and includes a structure having a mean particle size in the range of 20 - 300 microns.

The terms "extracellular matrix", "ECM" and "ECM material" are used interchangeably herein, and mean and include a collagen-rich substance that is found in between cells in mammalian tissue, and any material processed therefrom, e.g. decellularized ECM. According to the invention, the ECM material can be derived from a variety of mammalian tissue sources, selected from the group comprising small intestine submucosa (SIS), urinary bladder submucosa (UBS), stomach submucosa (SS), epithelium of mesodermal origin, i.e. mesothelial tissue, gastrointestinal extracellular matrix, i.e. small intestines, tissue surrounding growing bone, placental extracellular matrix, cardiac extracellular matrix, e.g., pericardium and/or myocardium. The ECM material can also comprise collagen from mammalian sources.

The terms "urinary bladder submucosa (UBS)", "small intestine submucosa (SIS)" and "stomach submucosa (SS)" also mean and include any UBS and/or SIS and/or SS material that includes the tunica mucosa (which includes the transitional epithelial layer and the tunica propria), submucosal layer, one or more layers of muscularis, and adventitia (a loose connective tissue layer) associated therewith.

The ECM material can also be derived from basement membrane of mammalian tissue/organs, including, urinary basement membrane (UBM), liver basement membrane (LBM), and amnion, chorion, allograft pericardium, allograft acellular dermis, amniotic membrane, Wharton's jelly, and combinations thereof.

Additional sources of mammalian basement membrane include, spleen, lymph nodes, salivary glands, prostate, pancreas and other secreting glands.

ECM material can also be derived from other sources, including, without limitation, collagen from plant sources and synthesized extracellular matrices, i.e. cell cultures.

The term "angiogenesis", as used herein, means a physiologic process involving the growth of new blood vessels from pre-existing blood vessels.

The term "neovascularization", as used herein, means and includes the formation of functional vascular networks that can be perfused by blood or blood components. Neovascularization includes angiogenesis, budding angiogenesis, intussuceptive angiogenesis, sprouting angiogenesis, therapeutic angiogenesis and vasculogenesis.

The terms "ECM-mimicking biomaterial", and "ECM-mimicking material" are used interchangeably herein, and mean and include a biocompatible and biodegradable biomaterial that induces neovascularization and bioremodeling of tissue *in vivo,* i.e. when disposed proximate damaged biological tissue. The term "ECM-mimicking" thus includes, ECM-mimicking polymeric biomaterial compositions; specifically, poly(glycerol sebacate) (PGS).

The terms "biologically active agent" and "biologically active composition" are used interchangeably *herein,* and mean and include agent that induces or modulates a physiological or biological process, or cellular activity, e.g., induces proliferation, and/or growth and/or regeneration of tissue.

The terms "biologically active agent" and "biologically active composition" thus mean and include, without limitation, the following growth factors: platelet derived growth factor (PDGF), epidermal growth factor (EGF), transforming growth factor alpha (TGF-alpha), transforming growth factor beta (TGF-beta), fibroblast growth factor - 2 (FGF-2), basic fibroblast growth factor (bFGF) vascular epithelial growth factor (VEGF), hepatocyte growth factor (HGF), insulin-like growth factor (IGF), nerve growth factor (NGF), platlet derived growth factor (PDGF), tumor necrosis factor alpha (TNA-alpha), and placental growth factor (PLGF).

The terms "biologically active agent" and "biologically active composition" also mean and include, without limitation, human embryonic stem cells, fetal cardiomyocytes, myofibroblasts, mesenchymal stem cells, autotransplated expanded cardiomyocytes, adipocytes, totipotent cells, pluripotent cells, blood stem cells, myoblasts, adult stem cells, bone marrow cells, mesenchymal cells, embryonic stem cells, parenchymal cells, epithelial cells, endothelial cells, mesothelial cells, fibroblasts, osteoblasts, chondrocytes, exogenous cells, endogenous cells, stem cells, hematopoietic stem cells, bone-marrow derived progenitor cells, myocardial cells, skeletal cells, fetal cells, undifferentiated cells, multi-potent progenitor cells, unipotent progenitor cells, monocytes, cardiac myoblasts, skeletal myoblasts, macrophages, capillary endothelial cells, xenogenic cells, allogenic cells, and post-natal stem cells. The (embryonic) stem cells are not totipotent human embryonic cells or cells obtained by destruction of embryos.

The terms "biologically active agent" and "biologically active composition" also mean and include, without limitation, the following biologically active agents (referred to interchangeably herein as a "protein", "peptide" and "polypeptide"): collagen (types I-V), proteoglycans, glycosaminoglycans (GAGs), glycoproteins, growth factors, cytokines, cell-surface associated proteins, cell adhesion molecules (CAM), angiogenic growth factors, endothelial ligands, matrikines, cadherins, immuoglobins, fibril collagens, non-fibrallar collagens, basement membrane collagens, multiplexins, small-leucine rich proteoglycans, decorins, biglycans, fibromodulins, keratocans, lumicans, epiphycans, heparin sulfate proteoglycans, perlecans, agrins, testicans, syndecans, glypicans, serglycins, selectins, lecticans, aggrecans, versicans, neurocans, brevicans, cytoplasmic domain-44 (CD-44), macrophage stimulating factors, amyloid precursor proteins, heparins, chondroitin sulfate B (dermatan sulfate), chondroitin sulfate A, heparin sulfates, hyaluronic acids, fibronectins, tenascins, elastins, fibrillins, laminins, nidogen/enactins, fibulin I, fibulin II, integrins, transmembrane molecules, thrombospondins, ostepontins, and angiotensin converting enzymes (ACE).

The terms "pharmacological agent", "active agent", "drug" and "active agent formulation" are used interchangeably herein, and mean and include an agent, drug, compound, composition of matter or mixture thereof, including its formulation, which provides some therapeutic, often beneficial, effect. This includes any physiologically or pharmacologically active substance that produces a localized or systemic effect or effects in animals, including warm blooded mammals, humans and primates; avians; domestic household or farm animals, such as cats, dogs, sheep, goats, cattle, horses and pigs; laboratory animals, such as mice, rats and guinea pigs; fish; reptiles; zoo and wild animals; and the like.

The terms "pharmacological agent", "active agent", "drug" and "active agent formulation" thus mean and include, without limitation, antibiotics, anti-arrhythmic agents, anti-viral agents, analgesics, steroidal anti-inflammatories, non-steroidal anti-inflammatories, anti-neoplastics, anti-spasmodics, modulators of cell-extracellular matrix interactions, proteins, hormones, growth factors, matrix metalloproteinases (MMPs), enzymes and enzyme inhibitors, anticoagulants and/or anti-thrombic agents, DNA, RNA, modified DNA and RNA, NSAIDs, inhibitors of DNA, RNA or protein synthesis, polypeptides, oligonucleotides, polynucleotides, nucleoproteins, compounds modulating cell migration, compounds modulating proliferation and growth of tissue, and vasodilating agents.

The terms "pharmacological agent", "active agent", "drug" and "active agent formulation" thus include, without limitation, atropine, tropicamide, dexamethasone, dexamethasone phosphate, betamethasone, betamethasone phosphate, prednisolone, triamcinolone, triamcinolone acetonide, fluocinolone acetonide, anecortave acetate, budesonide, cyclosporine, FK-506, rapamycin, ruboxistaurin, midostaurin, flurbiprofen, suprofen, ketoprofen, diclofenac, ketorolac, nepafenac, lidocaine, neomycin, polymyxin b, bacitracin, gramicidin, gentamicin, oyxtetracycline, ciprofloxacin, ofloxacin, tobramycin, amikacin, vancomycin, cefazolin, ticarcillin, chloramphenicol, miconazole, itraconazole, trifluridine, vidarabine, ganciclovir, acyclovir, cidofovir, ara-amp, foscarnet, idoxuridine, adefovir dipivoxil, methotrexate, carboplatin, phenylephrine, epinephrine, dipivefrin, timolol, 6-hydroxydopamine, betaxolol, pilocarpine, carbachol, physostigmine, demecarium, dorzolamide, brinzolamide, latanoprost, sodium hyaluronate, insulin, verteporfin, pegaptanib, ranibizumab, and other antibodies, antineoplastics, anti-VEGFs, ciliary neurotrophic factor, brain-derived neurotrophic factor, bFGF, Caspase-1 inhibitors, Caspase-3 inhibitors, α-Adrenoceptors agonists, NMDA antagonists, Glial cell line-derived neurotrophic factors (GDNF), pigment epithelium-derived factor (PEDF), and NT-3, NT-4, NGF, IGF-2.

The terms "pharmacological agent", "active agent", "drug" and "active agent formulation" further mean and include the following Class I - Class V antiarrhythmic agents: (Class la) quinidine, procainamide and disopyramide; (Class lb) lidocaine, phenytoin and mexiletine; (Class Ic) flecainide, propafenone and moricizine; (Class II) propranolol, esmolol, timolol, metoprolol and atenolol; (Class III) amiodarone, sotalol, ibutilide and dofetilide; (Class IV) verapamil and diltiazem) and (Class V) adenosine and digoxin.

The terms "pharmacological agent", "active agent", "drug" and "active agent formulation" further mean and include, without limitation, the following antiobiotics: aminoglycosides, cephalosporins, chloramphenicol, clindamycin, erythromycins, fluoroquinolones, macrolides, azolides, metronidazole, penicillins, tetracyclines, trimethoprim-sulfamethoxazole and vancomycin.

The terms "pharmacological agent", "active agent", "drug" and "active agent formulation" further include, without limitation, the following steroids: andranes (e.g., testosterone), cholestanes, cholic acids, corticosteroids (e.g., dexamethasone), estraenes (e.g., estradiol) and pregnanes (e.g., progesterone).

The terms "pharmacological agent", "active agent", "drug" and "active agent formulation" can further include one or more classes of narcotic analgesics, including, without limitation, morphine, codeine, heroin, hydromorphone, levorphanol, meperidine, methadone, oxycodone, propoxyphene, fentanyl, methadone, naloxone, buprenorphine, butorphanol, nalbuphine and pentazocine.

The terms "pharmacological agent", "active agent", "drug" and "active agent formulation" can further include one or more classes of topical or local anesthetics, including, without limitation, esters, such as benzocaine, chloroprocaine, cocaine, cyclomethycaine, dimethocaine/larocaine, piperocaine, propoxycaine, procaine/novacaine, proparacaine, and tetracaine/amethocaine. Local anesthetics can also include, without limitation, amides, such as articaine, bupivacaine, cinchocaine/dibucaine, etidocaine, levobupivacaine, lidocaine/lignocaine, mepivacaine, prilocaine, ropivacaine, and trimecaine. Local anesthetics can further include combinations of the above from either amides or esters.

The terms "anti-inflammatory" and "anti-inflammatory agent" are also used interchangeably herein, and mean and include a "pharmacological agent" and/or "active agent formulation", which, when a therapeutically effective amount is administered to a subject, prevents or treats bodily tissue inflammation i.e. the protective tissue response to injury or destruction of tissues, which serves to destroy, dilute, or wall off both the injurious agent and the injured tissues.

Anti-inflammatory agents thus include, without limitation, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, decanoate, deflazacort, delatestryl, depo-testosterone, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, mesterolone, methandrostenolone, methenolone, methenolone acetate, methylprednisolone suleptanate, momiflumate, nabumetone, nandrolone, naproxen, naproxen sodium, naproxol, nimazone, olsalazine sodium, orgotein, orpanoxin, oxandrolane, oxaprozin, oxyphenbutazone, oxymetholone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, stanozolol, sudoxicam, sulindac, suprofen, talmetacin, talniflumate, talosalate, tebufelone, tenidap, tenidap sodium, tenoxicam, tesicam, tesimide, testosterone, testosterone blends, tetrydamine, tiopinac, tixocortol pivalate, tolmetin, tolmetin sodium, triclonide, triflumidate, zidometacin, and zomepirac sodium.

The tenn "pharmacological composition", as used herein, means and includes a composition comprising a "pharmacological agent" and/or a "biologically active agent" and/or any additional agent or component identified herein.

The term "therapeutically effective", as used herein, means that the amount of the "pharmacological agent" and/or "biologically active agent" and/or "pharmacological composition" administered is of sufficient quantity to ameliorate one or more causes, symptoms, or sequelae of a disease or disorder. Such amelioration only requires a reduction or alteration, not necessarily elimination, of the cause, symptom, or sequelae of a disease or disorder.

The term "adolescent", as used herein, means and includes a mammal that is preferably less than three (3) years of age.

The terms "patient" and "subject" are used interchangeably herein, and mean and include warm blooded mammals, humans and primates; avians; domestic household or farm animals, such as cats, dogs, sheep, goats, cattle, horses and pigs; laboratory animals, such as mice, rats and guinea pigs; fish; reptiles; zoo and wild animals; and the like.

The term "comprise" and variations of the term, such as "comprising" and "comprises," means "including, but not limited to" and is not intended to exclude, for example, other additives, components, integers or steps.

The following disclosure is provided to further explain in an enabling fashion the best modes of performing one or more embodiments of the present invention. The disclosure is further offered to enhance an understanding and appreciation for the inventive principles and advantages thereof, rather than to limit in any manner the invention. The invention is defined solely by the claims as issued.

The present invention is directed to a biomaterial composition for use in treating damaged cardiovascular tissue as defined in the claim 1. Advantageous embodiments of the invention are characterized in the dependent claims.

As stated above, tissue prostheses comprise an ECM sheet member having at least one defined surface. In an insistance of the disclosure, the defined surface comprises a biomaterial composition coated surface.

In an instance of the disclosure, the tissue prostheses comprise a multi-sheet laminate structure. In an instance of the disclosure the multi-sheet laminate structure comprises a base ECM sheet member comprising an ECM material and a top biomaterial composition coated surface that is in communication with (i.e. in contact with) a non-coated surface of a second ECM sheet member.

In an instance of the disclosure, the multi-sheet laminate structure comprises a plurality of base ECM sheet members having a top biomaterial composition coated surface and a bottom surface, the bottom surface of each adjoining base ECM sheet member being in communication with a biomaterial composition coated surface of a base ECM sheet member, and a top ECM sheet layer having top and bottom surfaces, the bottom surface of which being in communication with a biomaterial composition coated surface of a base ECM sheet member.

In a preferred embodiment of the invention, the biomaterial composition comprises poly(glycerol sebacate) (PGS).

According to the invention, the particulate structures comprise a particulate ECM component comprising an ECM composition having at least one ECM material that is encased in a biomaterial composition.

The biomaterial composition similarly comprises an ECM-mimicking biomaterial composition comprising poly(glycerol sebacate) (PGS).

Applicant has found that PGS substantially reduces or eliminates dilation and/or delamination of the prosthesis structures, i.e. between ECM sheet members.

PGS also exhibits numerous beneficial biochemical actions or activities. The properties and beneficial actions resulting therefrom are discussed in detail below.

### PGS Physical Properties

PGS is a condensate of the non-immunogenic compositions glycerol (a simple sugar alcohol) and sebacic acid (a naturally occurring dicarboxylic acid), wherein, glycerol and sebacic acid are readily metabolized when proximate mammalian tissue. The non-immunogenic properties substantially limit the acute inflammatory responses typically associated with other "biocompatible" polymers, such as ePTFE (polytetrafluoroethylene), that are detrimental to bioremodeling and tissue regeneration.

The mechanical properties of PGS are substantially similar to that of biological tissue. Indeed, the value of the Young's modulus of PGS is between that of a ligament (in KPa range) and tendon (in GPa range). The strain to failure of PGS is also similar to that of arteries and veins (i.e. over 260% elongation).

The tensile strength of the PGS is at least 0.28 ± 0.004 MPa, The Young's modulus and elongation are at least 0.122 ± 0.0003 and at least 237.8± 0.64%, respectively. For applications requiring stronger mechanical properties and a slower biodegradation rate, PGS can be blended with poly(ε-caprolactone) PCL, i.e. a biodegradable elastomer.

### ECM Mimicking Properties/Actions

It has been established that PGS induces tissue remodeling and regeneration when administered proximate to damaged tissue, thus, mimicking the seminal regenerative properties of ECM and, hence, an ECM composition formed therefrom. The mechanism underlying this behavior is deemed to be based on the mechanical and biodegradation kinetics of the PGS. See Sant, et al., Effect of Biodegradation and de novo Matrix Synthesis on the Mechanical Properties of VIC-seeded PGS-PCL scaffolds, Acta. Biomater., vol. 9(4), pp. 5963-73 (2013).

In some embodiments, the ECM-mimicking biomaterial composition further comprises one of the aforementioned ECM materials.

In some embodiments of the invention, the ECM-mimicking biomaterial composition comprises PGS and poly(ε-caprolactone) (PCL). According to the invention, the addition of PCL to the ECM-mimicking biomaterial composition enhances the structural integrity and modulates the degradation of the composition.

In some embodiments, the ECM-mimicking biomaterial composition comprises poly(glycerol sebacate) acrylate (PGSA), which, can be crosslinked and/or cured via the combination of a photoinitiator and radiation.

Suitable photoinitiators for radiation induced crosslinking comprise, without limitation, 2-hydroxy-1-[4-hydroxyethoxy)phenyl]-2-methyl-1-propanone(D 2959, Ciba Geigy), 2,2-dimethoxy-2-phenylacetophenone, titanocenes, fluorinated diaryltitanocenes, iron arene complexes, manganese decacarbonyl, methylcyclopentadienyl manganese tricarbonyl and any organometallatic photoinitiator that produces free radicals or cations.

Suitable radiation wavelengths for crosslinking and/or curing the ECM-mimicking biomaterial composition comprise, without limitation, visible light; particularly, radiation in the range of approximately 380-750 nm, and ultraviolet (UV) light, particularly, radiation in the range of 10-400 nm, which includes extreme UV (10-121 nm), vacuum UV (10-200 nm), hydrogen lyman α-UV (121-122 nm), Far UV (122-200 nm), Middle UV (200-300 nm), Near UV (300-400 nm), UV-C (100-280 nm), UV-B (280-315 nm) and UV-A (315-400 nm) species of UV light.

In some embodiments, the ECM-mimicking biomaterial composition comprises a co-polymer of PGSA and polyethylene glycol (PEG) diacrylate.

Preferably, the ratio of PGSA to PEG diacrylate used when developing the photocured PGSA is proportional to the physical strength of the biomaterial composition, wherein a ratio of PGSA to PEG diacrylate in the range of 95:05-50:50 comprises a Young's modulus in the range of approximately 0.5-20 MPa respectively.

### ECM Materials

In a preferred embodiment of the invention, the ECM material(s) comprise a decellularized ECM material from a mammalian tissue source. According to the invention, the mammalian tissue sources include, the small intestine, liver, placenta, heart, bladder, and any fetal tissue from any mammalian organ.

The ECM material can thus comprise, small intestine submucosa (SIS), urinary bladder submucosa (UBS), stomach submucosa (SS), gastrointestinal extracellular matrix, i.e. small intestines, placental extracellular matrix, epithelium of mesodermal origin, i.e. mesothelial tissue, cardiac extracellular matrix, e.g., pericardium and/or myocardium.

The ECM material can also comprise collagen from mammalian sources.

In a preferred embodiment, the mammalian tissue source comprises an adolescent mammalian tissue source, i.e. an adolescent mammal, such as a piglet, which is preferably less than three (3) years of age.

In a preferred embodiment, the ECM material is decellularized and, hence, remodelable. According to the invention, the ECM material can be decellularized by various conventional means. In a preferred embodiment, the ECM material is decellularized via one of the unique Novasterilis processes disclosed in U.S. Pat. No. 7,108,832 and U.S. Pat. App. No. 13/480,204.

According to the invention, the ECM material can be formed into a particulate to form a particulate ECM component of the invention and fluidized, as described in U.S. Pat. Nos. 5,275,826, 6,579,538 and 6,933,326, to form an ECM composition of the invention.

According to the invention, various conventional means can be employed to form a particulate ECM material and, hence, component. In some embodiments, the ECM material is formed into a sheet, fluidized (or hydrated), if necessary, frozen and ground.

In some embodiments of the invention, the ground ECM material is subsequently filtered to achieve a desired particulate size. In a preferred embodiment, the ECM material has a particulate size in the range of about 20 microns to about 300 microns.

In a preferred embodiment of the invention, a plurality of the particulate structures is employed to form biomaterial compositions of the invention. According to the invention, the biomaterial compositions can comprise mixed liquids, mixed emulsions, mixed gels, mixed pastes, or mixed solid particulates. The liquid or semi-solid components of the biomaterial compositions can also comprise various concentrations.

In some embodiments of the invention, the biomaterial compositions thus include a suitable buffer solution, such as saline.

Preferably, the concentration of the liquid or semi-solid components of the biomaterial compositions is in the range of about 0.001 mg/ml to about 200 mg/ml. Suitable concentration ranges thus include, without limitation: about 5 mg/ml to about 150 mg/ml, about 10 mg/ml to about 125 mg/ml, about 25 mg/ml to about 100 mg/ml, about 20 mg/ml to about 75 mg/ml, about 25 mg/ml to about 60 mg/ml, about 30 mg/ml to about 50 mg/ml, and about 35 mg/ml to about 45 mg/ml and about 40 mg/ml. to about 42 mg/ml.

The noted concentration ranges are, however, merely exemplary and not intended to be exhaustive or limiting. It is understood that any value within any of the listed ranges is deemed a reasonable and useful value for a concentration of a liquid or semi-solid component of a biomaterial composition of the invention.

As stated above, in an instance of the disclosure, the ECM composition and/or biomaterial composition and, hence, tissue prostheses and/or particulate structures formed therefrom or therewith further comprise at least one additional biologically active agent or composition, i.e. an agent that induces or modulates a physiological or biological process, or cellular activity, e.g., induces proliferation, and/or growth and/or regeneration of tissue.

In an instance of the disclosure, the biologically active agent is similarly derived from an adolescent mammal, i.e. a mammal less than three (3) years of age.

Suitable biologically active agents include any of the aforementioned biologically active agents, including, without limitation, the aforementioned cells and proteins.

In some embodiments of the invention, the biomaterial composition of the present invention comprises a growth factor selected from the group comprising transforming growth factor-beta (TGF-β), basic fibroblast growth factor (bFGF) and vascular epithelial growth factor (VEGF).

In an instance of the disclosure, the biologically active agent comprises a protein selected from the group comprising proteoglycans, glycosaminoglycans (GAGs), glycoproteins, heparins, chondroitin sulfate B (dermatan sulfate), chondroitin sulfate A, heparin sulfates, and hyaluronic acids.

In an instance of the disclosure, the protein comprises a cytokine selected from the group comprising a stem cell factor (SCF), stromal cell-derived factor-1 (SDF-1), granulocyte macrophage colony-stimulating factor (GM-CSF), interferon gamma (IFN-gamma), interleukin-3, interleukin-4, interleukin-8, interleukin-10, interleukin-13, leukemia inhibitory factor (LIF), amphiregulin, thrombospondin 1, thrombospondin 2, thrombospondin 3, thrombospondin 4, thrombospondin 5, and angiotensin converting enzyme (ACE).

In an instance of the disclosure, the ECM composition and/or biomaterial composition and, hence, tissue prostheses and/or particulate structures formed therefrom or therewith further comprise at least one pharmacological agent or composition (or drug), i.e. an agent or composition that is capable of producing a desired biological effect *in vivo*, e.g., stimulation or suppression of apoptosis, stimulation or suppression of an immune response, etc.

Suitable pharmacological agents and compositions include any of the aforementioned agents, including, without limitation, antibiotics, anti-viral agents, analgesics, steroidal anti-inflammatories, non-steroidal anti-inflammatories, anti-neoplastics, anti-spasmodics, modulators of cell-extracellular matrix interactions, proteins, hormones, enzymes and enzyme inhibitors, anticoagulants and/or anti-thrombic agents, DNA, RNA, modified DNA and RNA, NSAIDs, inhibitors of DNA, RNA or protein synthesis, polypeptides, oligonucleotides, polynucleotides, nucleoproteins, compounds modulating cell migration, compounds modulating proliferation and growth of tissue, and vasodilating agents.

In an instance of the disclosure, the pharmacological agent comprises one of the aforementioned anti-inflammatory agents.

In an instance of the disclosure, the pharmacological agent comprises a statin, i.e. a HMG-CoA reductase inhibitor. Suitable statins include, without limitation, atorvastatin (Lipitor®), cerivastatin, fluvastatin (Lescol®), lovastatin (Mevacor®, Altocor®, Altoprev®), mevastatin, pitavastatin (Livalo®, Pitava®), pravastatin (Pravachol®, Selektine®, Lipostat®), rosuvastatin (Crestor®), and simvastatin (Zocor®, Lipex®). Several actives comprising a combination of a statin and another agent, such as ezetimbe/simvastatin (Vytorin®), are also suitable.

Applicant has found that the noted statins exhibit numerous beneficial properties that provide several beneficial biochemical actions or activities. Among the beneficial biochemical actions, Applicant has found that when a statin is added to ECM (wherein a statin augmented ECM member is formed) and the statin augmented ECM member is administered to damaged tissue, the statin interacts with the cells recruited by the ECM, wherein the statin augmented ECM member modulates inflammation of the damaged tissue by modulating several significant inflammatory processes, including restricting expression of monocyte chemoattractant protein-1 (MCP-1) and chemokine (C-C) motif ligand 2 (CCR2).

Further beneficial actions are discussed in detail in Applicant's Co-Pending Application Nos. 13/328,287, filed on December 16, 2011, 13/373,569, filed on September 24, 2012 and 13/782,024, filed on March 1, 2013.

In an instance of the disclosure, the ECM member and, hence, tissue prosthesis formed therefrom further comprises at least one anchoring mechanism, such as disclosed in Co-pending Application Nos. 1 3/782,024 and 13/686,131.

In an instance of the disclosure, upon disposing a tissue prosthesis and/or particulate structure of the invention proximate damaged or diseased biological tissue, "modulated healing" is effectuated.

The term "modulated healing", as used herein, and variants of this language generally refer to the modulation (e.g., alteration, delay, retardation, reduction, etc.) of a process involving different cascades or sequences of naturally occurring tissue repair in response to localized tissue damage or injury, substantially reducing their inflammatory effect. Modulated healing, as used herein, includes many different biologic processes, including epithelial growth, fibrin deposition, platelet activation and attachment, inhibition, proliferation and/or differentiation, connective fibrous tissue production and function, angiogenesis, and several stages of acute and/or chronic inflammation, and their interplay with each other.

For example, the tissue prosthesis and/or particulate structure is specifically formulated (or designed) to alter, delay, retard, reduce, and/or detain one or more of the phases associated with healing of damaged tissue, including, but not limited to, the inflammatory phase (e.g., platelet or fibrin deposition), and the proliferative phase.

Herein, "modulated healing" refers to the ability of a tissue prosthesis and/or particulate structure to alter a substantial inflammatory phase (e.g., platelet or fibrin deposition) at the beginning of the tissue healing process. As used herein, the phrase "alter a substantial inflammatory phase" refers to the ability of a tissue prosthesis and/or particulate structure to substantially reduce the inflammatory response at an injury site.

In such an instance, a minor amount of inflammation may ensue in response to tissue injury, but this level of inflammation response, e.g., platelet and/or fibrin deposition, is substantially reduced when compared to inflammation that takes place in the absence of a tissue prosthesis as described herein.

In some instances of the disclosure, "modulated healing" refers to the ability of a tissue prosthesis and/or particulate structure to induce host cell and/or tissue proliferation, bioremodeling, including neovascularization, e.g., vasculogenesis, angiogenesis, and intussusception, and regeneration of tissue structures with site-specific structural and functional properties when disposed proximate damaged tissue.

In some instances of the disclosure, the term "modulated healing" thus means and includes the ability of tissue prosthesis and/or particulate structure to modulate inflammation and/or induce host cell and/or tissue proliferation and bioremodeling when disposed proximate damaged tissue.

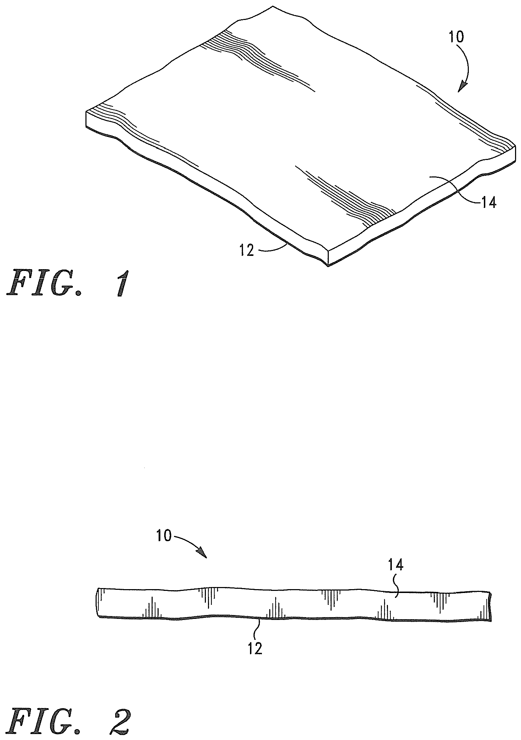

Referring now to Figs. 1 and 2, there is shown an ECM sheet member. As illustrated in Fig. 2, the ECM sheet member 10 comprises a biomaterial composition coated surface 14 and a bottom surface 12.

The ECM sheet member 10 can further comprise top and bottom biomaterial composition coated surfaces.

As indicated above, ECM sheet member 10 comprises a decellularized ECM material. As also indicated above, preferably, the ECM material is derived from an adolescent mammal, i.e. a mammal less than three (3) years of age.

The ECM sheet member 10, and, hence tissue prosthesis formed therefrom can comprise various shapes and dimensions to accommodate various applications.

In some instances of the disclosure, the ECM sheet member 10 (and, hence, ECM material thereof) and/or biomaterial composition coated surface 14 further comprises at least one additional biologically active agent or composition, i.e. an agent that induces or modulates a physiological or biological process, or cellular activity, e.g., induces proliferation, and/or growth and/or regeneration of tissue.

Suitable biologically active agents include any of the aforementioned biologically active agents, including, without limitation, the aforementioned cells, growth factors and proteins.

In some instances of the disclosure, the ECM member 10 (and, hence, ECM material thereof) biomaterial composition coated surface 14 further comprises at least one pharmacological agent or composition (or drug), i.e. an agent or composition that is capable of producing a desired biological effect *in vivo,* e.g., stimulation or suppression of apoptosis, stimulation or suppression of an immune response, etc.

Suitable pharmacological agents and compositions include any of the aforementioned agents, including, without limitation, antibiotics, anti-viral agents, analgesics, and anti-inflammatories.

In some instances of the disclosure, the pharmacological agent comprises a statin.

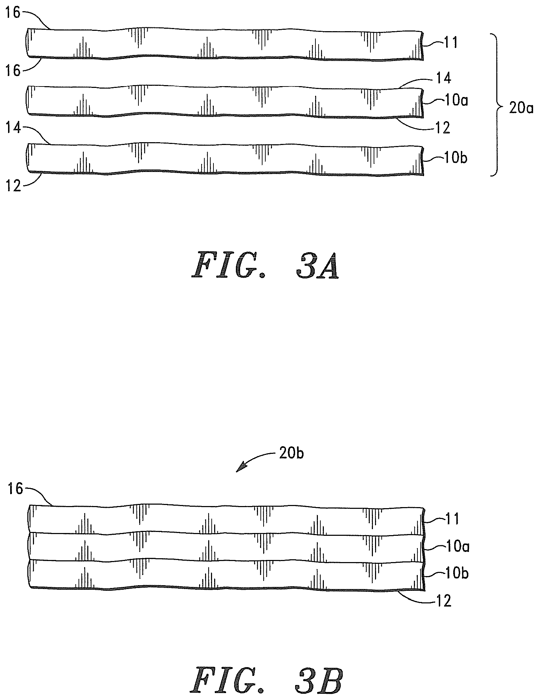

Referring now to Fig. 3A, there is shown a multi-sheet pre-laminate structure 20a that can be employed to construct a multi-sheet tissue prosthesis. In the illustrated example, the pre-laminate structure 20a comprises first and second ECM sheet members 10a, 10b, each sheet member 10a, 10b having a top biomaterial composition coated surface 14 and a bottom surface 12. The pre-laminate structure 20a further comprises a third ECM sheet member 11 having top and bottom surfaces 16.

Referring now to Fig. 3B, there is shown of a multi-sheet tissue prosthesis 20b that is formed from the pre-laminate structure shown in Fig. 3A. As illustrated in Fig. 3B, the bottom surface 12 of the first ECM sheet member 10a is in communication with a biomaterial composition coated surface 14 of the adjoining second ECM sheet member 10b, and the bottom surface 16 of the third ECM sheet member 11 is in communication with the biomaterial composition coated surface 14 of the first ECM sheet member 10a. The resultant structure thus comprises three layer laminated ECM structure with a non-coated top and bottom surface.

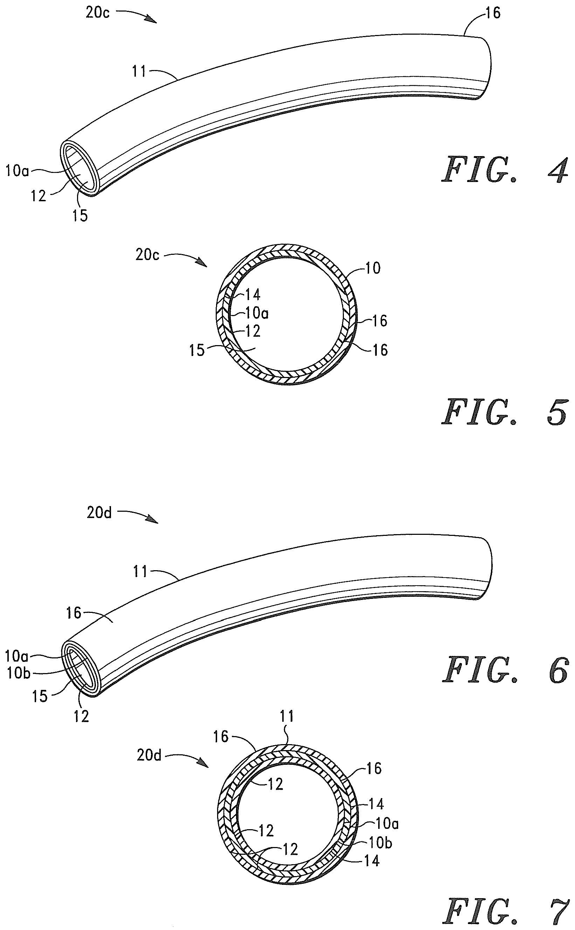

Referring now to Figs. 4 and 5, there is shown another example of a tissue prosthesis. As illustrated in Fig. 4, the prosthesis 20c comprises tubular member having a lumen 15 that extends therethrough.

As illustrated in Fig. 5, the prosthesis 20c comprises a bottom or base ECM sheet member 10a that similarly includes a top biomaterial composition coated surface 14 and a bottom surface 12, and an adjoining top ECM sheet member 11 having top and bottom surfaces 16. As further illustrated in Fig. 5, the bottom surface 16 of the ECM sheet member 11 is in communication with the top biomaterial composition coated surface 14 of the base ECM sheet member 10a.

Referring now to Figs. 6 and 7, there is shown another example of a tissue prosthesis. As illustrated in Fig. 6, the prosthesis 20d similarly comprises a tubular member having a lumen 15 that extends therethrough.

As illustrated in Fig. 7, the prosthesis 20d comprises first and second ECM sheet members 10a, 10b having biomaterial composition coated surfaces 14 and bottom surfaces 12; the bottom surface 12 of the first ECM member 10a being in communication with the biomaterial composition coated surface 14 of the second adjoining ECM sheet member 10b (or sheet layer), and a top ECM sheet member 11 having top and bottom surfaces 16. As further illustrated in Fig. 7, the bottom surface 16 of the ECM sheet member 11 is in communication with the top biomaterial composition coated surface 14 of the first ECM sheet member 10a.

The multi-sheet tissue prostheses, including prostheses 20b, 20c and 20d described above, can be formed in any ECM sheet member order; provided, that the surfaces of the top and bottom ECM sheet members are preferably non-coated surfaces.

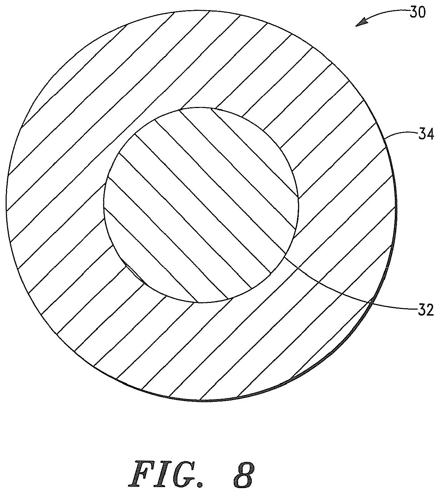

Referring now to Fig. 8, there is shown one embodiment of a particulate structure of the invention. As illustrated in Fig. 8, the particulate structure 30 comprises a particulate component 32 comprising a first composition that is encased in a second composition 34.

As indicated above, according to the invention, the first composition of the particulate component 32 can comprise one of the aforementioned ECM compositions and the second composition 34 can comprise one of the aforementioned biomaterial compositions. The first composition can also comprise a biomaterial composition composition and the second composition 34 can comprise an ECM composition.

In a preferred embodiment of the invention, a plurality of the particulate structures shown in Fig. 1 are employed to form a biomaterial composition for treating damaged tissue.

In some embodiments, the biomaterial composition thus comprises a plurality of particulate structures comprising a particulate ECM component comprising an ECM composition, the ECM composition comprising at least one ECM material, each of said plurality of particulate ECM components being encased in a biomaterial composition, the biomaterial composition being configured to induce modulated healing when delivered to damaged biological tissue.

As will readily be appreciated by one having ordinary skill in the art, the prosthetic valves described herein provides numerous advantages compared to prior art prosthetic valves. Among the advantages are the following:
- The provision of tissue prostheses that substantially reduce or eliminate (i) the risk of thrombosis, (ii) intimal hyperplasia after intervention in a vessel, (iii) the harsh biological responses associated with conventional polymeric and metal prostheses, (iv) the formation of biofilm, inflammation and infection and (v) delamination.
- The provision of tissue prostheses and particulate structures that can be effectively employed to treat, reconstruct, replace and improve biological functions or promote the growth of new cardiovascular tissue in a cardiovascular structure.
- The provision of tissue prostheses and particulate structures that induce host tissue proliferation, bioremodeling and regeneration of new tissue and tissue structures with site-specific structural and functional properties.
- The provision of tissue prostheses and particulate structures that are capable of administering a pharmacological agent to host tissue and, thereby produce a desired biological and/or therapeutic effect.

## Claims

1. A biomaterial composition for use in treating damaged cardiovascular tissue, comprising:
a plurality of particulate structures and a liquid buffer solution, each of said plurality of particulate structures comprising a particulate component that is fully encased in a biomaterial coating,
said particulate component comprising a size in the range of 20-300 microns,
said particulate component further comprising an extracellular matrix (ECM) composition,
said ECM composition comprising acellular ECM from a mammalian tissue source,
said biomaterial coating comprising poly(glycerol sebacate) (PGS).

2. The biomaterial composition for use according to Claim 1, wherein said liquid buffer solution comprises saline.

3. The biomaterial composition for use according to Claim 1, wherein said mammalian tissue source is selected from the group consisting of small intestine submucosa (SIS), urinary bladder submucosa (UBS), urinary basement membrane (UBM), liver basement membrane (LBM), stomach submucosa (SS), mesothelial tissue, placental tissue and cardiac tissue.

4. The biomaterial composition for use according to Claim 1, wherein said ECM composition further comprises a growth factor selected from the group consisting of a basic fibroblast growth factor (bFGF), transforming growth factor-beta (TGF-β) and vascular epithelial growth factor (VEGF).

## Patentansprüche

1. Biomaterialzusammensetzung zur Verwendung in der Behandlung von geschädigtem kardiovaskulärem Gewebe, umfassend:
eine Vielzahl von partikulären Strukturen und eine flüssige Pufferlösung, wobei jede der Vielzahl von partikulären Strukturen eine partikuläre Komponente umfasst, die vollständig in einer Biomaterialbeschichtung eingeschlossen ist,
wobei die partikuläre Komponente eine Größe im Bereich von 20-300 Mikrometern aufweist,
wobei die partikuläre Komponente ferner eine extrazelluläre Matrix (ECM)-Zusammensetzung umfasst, wobei die ECM-Zusammensetzung azelluläre ECM aus einer Säugetiergewebequelle umfasst,
wobei die Biomaterialbeschichtung Poly(glycerinsebacat) (PGS) umfasst.

2. Biomaterialzusammensetzung zur Verwendung nach Anspruch 1, wobei die flüssige Pufferlösung Salzlösung umfasst.

3. Biomaterialzusammensetzung zur Verwendung nach Anspruch 1, wobei die Säugetiergewebequelle ausgewählt ist aus der Gruppe bestehend aus Dünndarm-Submukosa (SIS), Harnblasen-Submukosa (UBS), Harn-Basalmembran (UBM), Leber-Basalmembran (LBM), Magen-Submukosa (SS), Mesothelgewebe, Plazentagewebe und Herzgewebe.

4. Biomaterialzusammensetzung zur Verwendung nach Anspruch 1, wobei die ECM-Zusammensetzung ferner einen Wachstumsfaktor umfasst, der aus der Gruppe ausgewählt ist, die aus einem basischen Fibroblasten-Wachstumsfaktor (bFGF), transformierendem Wachstumsfaktor-beta (TGF-β) und vaskulärem epithelialen Wachstumsfaktor (VEGF) besteht.

## Revendications

1. Composition de biomatériau à utiliser pour traiter un tissu cardiovasculaire endommagé, comprenant:
une pluralité de structures particulaires et une solution tampon liquide, chacune de ladite pluralité de structures particulaires comprenant un composant particulaire qui est entièrement enveloppé dans un revêtement de biomatériau,
ledit composant particulaire comprenant une taille dans la plage de 20 à 300 microns,
ledit composant particulaire comprenant en outre un composant de matrice extracellulaire (ECM),
ladite composition ECM comprenant une ECM acellulaire provenant d'une source de tissu de mammifère,
ledit revêtement de biomatériau comprenant du poly(sébacate de glycérol) (PGS).

2. Composition de biomatériau à utiliser selon la revendication 1, dans laquelle ladite solution tampon liquide comprend une solution saline.

3. Composition de biomatériau à utiliser selon la revendication 1, dans laquelle ladite source de tissu de mammifère est choisie dans le groupe comprenant la sous-muqueuse de l'intestin grêle (SIS), la sous-muqueuse de la vessie (UBS), la membrane basale urinaire (UBM), la membrane basale du foie (LBM), la sous-muqueuse de l'estomac (SS), le tissu mésothélial, le tissu placentaire et le tissu cardiaque.

4. Composition de biomatériau à utiliser selon la revendication 1, dans laquelle ladite composition ECM comprend en outre un facteur de croissance choisi dans le groupe comprenant un facteur de croissance fibroblastique basique (bFGF), un facteur de croissance transformant bêta (TGF-R) et un facteur de croissance épithéliale vasculaire (VEGF).
